# EUROPEAN PATENT SPECIFICATION

(11) **EP 0 630 408 B1**
(45) Date of publication and mention of the grant of the patent: **02.01.2003**
(21) Application number: 93907261.7
(22) Date of filing: 04.03.1993
(51) Int. Cl.: C12N 15/80, C12N 15/53, C12N 9/08, C12N 1/15

(54) **PRODUCTION OF $i(ASPERGILLUS NIGER CATALASE-R)**
HERSTELLUNG VON -I(ASPERGILLUS NIGER KATALASE-R)
PRODUCTION DE LA CATALASE-R D'ASPERGILLUS NIGER

(30) Priority: 04.03.1992 US 846181; 04.03.1992 US 845989
(43) Date of publication of application: 28.12.1994
(73) Proprietor: GENENCOR INTERNATIONAL, INC., Palo Alto, California 94304-1013 (US)
(72) Inventor: BERKA, Randy, M., Davis , CA 95616 (US); FOWLER, Timothy, Redwood City, CA 94061 (US); REY, Michael, W., Davis, CA 95616 (US)
(74) Representative: Brandes, Jürgen, Dr.
(86) International application number: US9302020
(87) International publication number: WO93018166

(56) References cited:
- WO-A-86/06097
- DATABASE WPIL Section Ch, Week 7506, Derwent Publications Ltd., London, GB; Class D16, AN 75-10203W & SU,A,410 083 (CHEM SCI INST AS KAZAKH)
- JOURNAL OF BIOCHEMISTRY. vol. 92, no. 5, 1982, TOKYO JP pages 1449 - 1456 KYOKO KIKUCHI-TORII ET AL. 'Properties of Aspergillus niger catalase.'
- CHEMICAL ABSTRACTS, vol. 90, no. 3, 15 January 1979, Columbus, Ohio, US; abstract no. 18127q, GRUFT, H. ET AL. 'Properties of a unique catalase isolated from Aspergillus niger.' page 227 & CAN. J. BIOCHEM. vol. 56, no. 9, 1978, pages 916-919

## Description

### Field of the Invention:

The invention relates to the application of genetic engineering techniques to create novel strains of *A. niger* which produce high levels of an endogenous catalase enzyme (*catR* gene product, catalase-R) while generating minimal sodium gluconate waste material. Specifically, high levels of catalase-R are generated through replacement of the endogenous *catR* gene promoter with the *A. niger* glucoamylase (*glaA*) gene promoter which results in not only higher levels of catalase-R, but also eliminates the requirement for hydrogen peroxide to act as an inducer for catalase synthesis, and deletion of the endogenous glucose oxidase (*goxA*) gene greatly reduces the level of sodium gluconate waste product, thereby minimizing the need for expensive waste handling.

### Background of the Invention:

Catalases [hydrogen peroxide: hydrogen peroxide oxidoreductases (EC 1.11.1.6)] are enzymes which catalyze the conversion of hydrogen peroxide (H₂O₂) to oxygen (O₂) and water (H₂O) according to the following formula:

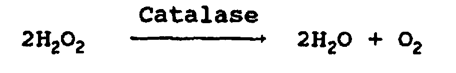

These ubiquitous enzymes have been purified from a variety of animal tissues, plants and microorganisms (Chance and Maehly 1955 Methods Enzymol. **2:** 764-791; Jones and Wilson 1978 in H. Sigel (ed.), *Metal Ions in Biological Systems,* Vol. 7, Marcel Dekker Inc., New York). Nearly all forms of the enzyme which have been characterized consist of four polypeptide subunits, each having a molecular weight of 50,000 to 60,000 and containing one protohemin prosthetic group per subunit (Wasserman and Hultin 1981 Arch. Biochem. Biophys. **212:** 385-392; Hartig and Ruis 1986 Eur. J. Biochem. **160:** 487-490). Bovine liver catalase has been the most extensively studied variety of this enzyme [Schonbaum and Chance 1976 in *The* *Enzymes* (P.D. Boyer, ed.) 3rd edn., vol. 13, pp. 363-408, Academic Press, New York]. The complete amino acid sequence and three dimensional structure of bovine liver catalase are known (Schroeder, et al., 1982 Arch. Biochem. Biophys. **214:** 397-412; Murthy, et al., 1981 J. Mol. Biol. **152:** 465-499).

Although less well-studied from a biochemical and biophysical standpoint, catalases from filamentous fungi have several characteristics that distinguish them from their mammalian counterparts. While similar in subunit number and heme content, fungal catalases are substantially larger molecules than those from other organisms, having subunit molecular weights ranging from 80,000 to 97,000 (Vainshtein, et al., 1986 J. Mol. Biol. **188:** 63-72; Jacob and Orme-Johnson 1979 Biochem. **18**: 2967-2975; Jones, et al., 1987 Biochim. Biophys. Acta **913**: 395-398). More importantly, catalases from fungi such as *Aspergillus niger* are more stable than beef liver catalase to proteolysis and to inactivation by glutaraldehyde, SDS, and have lower affinity for catalase inhibitors such as cyanide, azide and fluoride (Wasserman and Hultin 1981 Arch. Biochem. Biophys. **212**: 385-392). In addition, *A. niger* catalase is significantly more stable than beef liver catalase when subjected to extremes of pH, hydrogen peroxide, and temperature (Scott and Hammer 1960 Enzymologia **22**: 229-237). Although fungal catalases offer stability advantages, the corresponding mammalian enzymes such as beef liver catalase appear to have higher catalytic activity (Gruft, et al., 1978; Kikuchi-Torii, et al., 1982). However, since enzyme stability is an important factor in the biotechnological utilization of enzymes, there has been considerable interest in the use of fungal catalases, especially for applications involving neutralization of high concentrations of hydrogen peroxide. Vasudevan and Weiland (1990 Biotechnol. Bioeng. **36**: 783-789) observed that the rate of deactivation in H₂O₂ was at least an order of magnatude lower for *A. niger* catalase than for beef liver catalase. The differences in stability of these two enzymes can probably be attributed to differences in structural characteristics and composition of the proteins [Vasudevan and Weiland 1990 Biotechnol. Bioeng. **36:** 783-789].

Catalase preparations from *A. niger* are sold commercially for diagnostic enzyme kits, for the enzymatic production of sodium gluconate from glucose, for the neutralization of H₂O₂ waste, and for the removal of H₂O₂ and/or generation of O₂ in foods and beverages. Traditionally, beef liver catalase has been the preferred enzyme for diagnostic purposes and for pharmaceutical-related applications (e.g., contact-lens cleaning/disinfection/H₂O₂ neutralization). However, recent outbreaks of a slow-virus disease known as BSE (bovine spongiform encephalopathy) in European cattle herds and fear that this disease might be spread to man [Dealler and Lacey 1991 Nutr. Health (Bicester) **7:** 117-134; Dealler and Lacey 1990 Food Microbiol. **7:** 253-280] have aroused interests in finding alternatives to beef liver catalase for most industrial applications.

Little information has been published regarding the regulation of catalase synthesis in *A. niger*. However, it has been observed that catalase is produced in response to the generation of H₂O₂ during growth of the organism on glucose or fatty acids. For example, during the metabolism of glucose, H₂O₂ is formed by oxidation of the sugar to give gluconate. This reaction is catalyzed by the enzyme glucose oxidase:

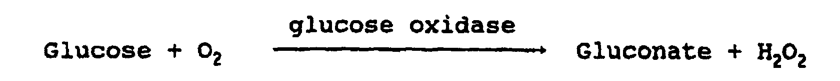

Cellular metabolism of fatty acids, which occurs in specialized organelles known as peroxisomes, also yields H₂O₂ which induces the formation of catalase. However, in a distantly related fungus (yeast), *Saccharomyces cerevisiae*, a specific catalase is induced during growth on fatty acids. This catalase, termed catalase-A (atypical), is localized chiefly in peroxisomes where fatty acid oxidation occurs. A second *S. cerevisiae* enzyme, catalase-T (typical) is a soluble cytoplasmic enzyme which is synthesized in response to a variety of other metabolic and environmental stresses. These two yeast catalases are the products of two different nuclear genes, designated *CTA1* and *CTT1*. Similarly, two catalase genes have been isolated from *A. niger* (Genencor International, Inc., unpublished). The *A. niger catA* gene, cloned by cross-hybridization to the yeast *CTA1* gene, encodes a catalase enzyme which is induced primarily during growth on fatty acids and is presumably peroxisomal. This enzyme (catalase-A) is not of commercial importance at this time, however, a second cloned *A. niger* catalase gene, designated as *catR*, encodes a soluble cytoplasmic enzyme (catalase-R) which represents the major activity in commercial catalase preparations.

Because of the obvious commercial interest in *A. niger* catalases, it would be desirable to obtain *A. niger* strains which produce increased levels of the catR gene product. Furthermore, it would be a significant advantage to effect high levels of catalase synthesis without the need to generate hydrogen peroxide as an inducer. Concomitant with the generation of hydrogen peroxide is the formation of sodium gluconate which represents a waste disposal problem. Thus, it is also highly desirable to minimize the production of gluconate in large scale fermentations with catalase production strains of *A. niger.* This invention discloses a solution for simultaneously accomplishing all of these objectives.

### Summary of the Invention:

It has been discovered that it is possible to increase the expression of catalase-R (*catR* gene product) without the need to supply hydrogen peroxide as an inducer of catalase synthesis. Simultaneously, it was discovered that elimination of glucose oxidase gene expression (by *goxA* gene deletion) minimizes the generation of gluconate waste material, thereby circumventing the need for expensive waste treatment processes.

The invention includes a gene encoding *Aspergillus niger* catalase-R (*catR* gene) to which promoter and terminator elements of the *A. niger* glucoamylase (*glaA*) gene were functionally attached. Concomitantly, the coding region of the *A. niger* glucose oxidase (*goxA*) gene was destroyed using a targeted gene replacement strategy. The invention also includes a transformed *A. niger* organism which is capable of expressing high levels of catalase-R without hydrogen peroxide induction. This organism contains a functional expression unit comprising the *catR* gene, to which the *A. niger glaA* gene promoter and terminator sequences have been functionally attached.

The inventors also disclose a method for producing high levels of catalase-R comprising growth of transformed *A. niger* cells which contain chromosomally integrated copies of the *catR* gene under operational control of the *A. niger glaA* promoter.

### Figures:

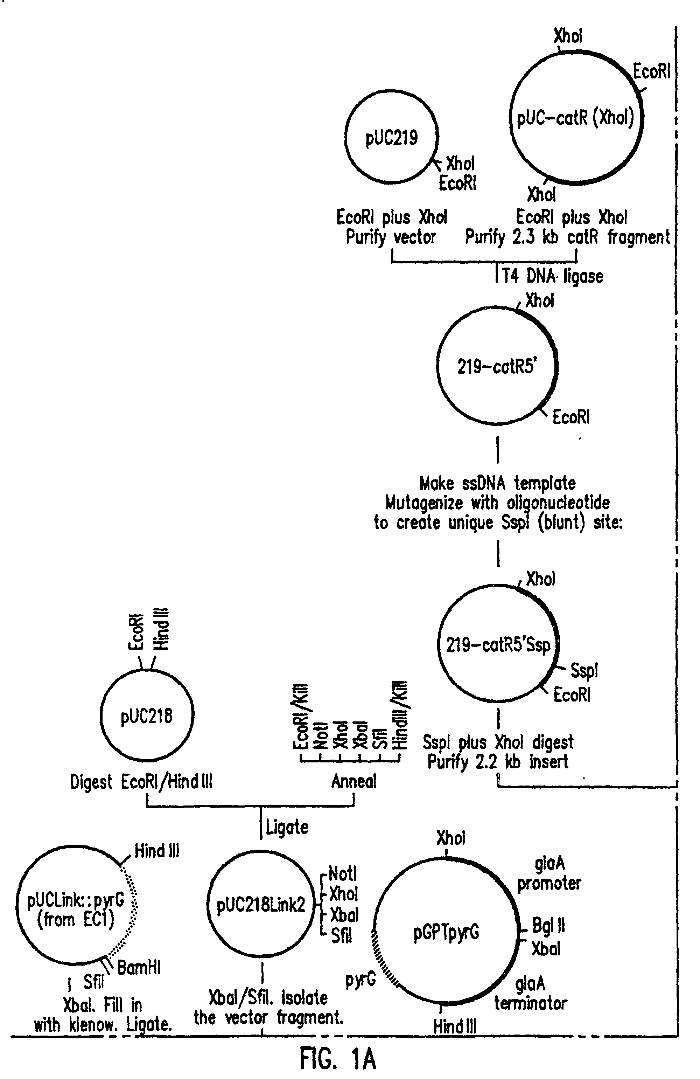

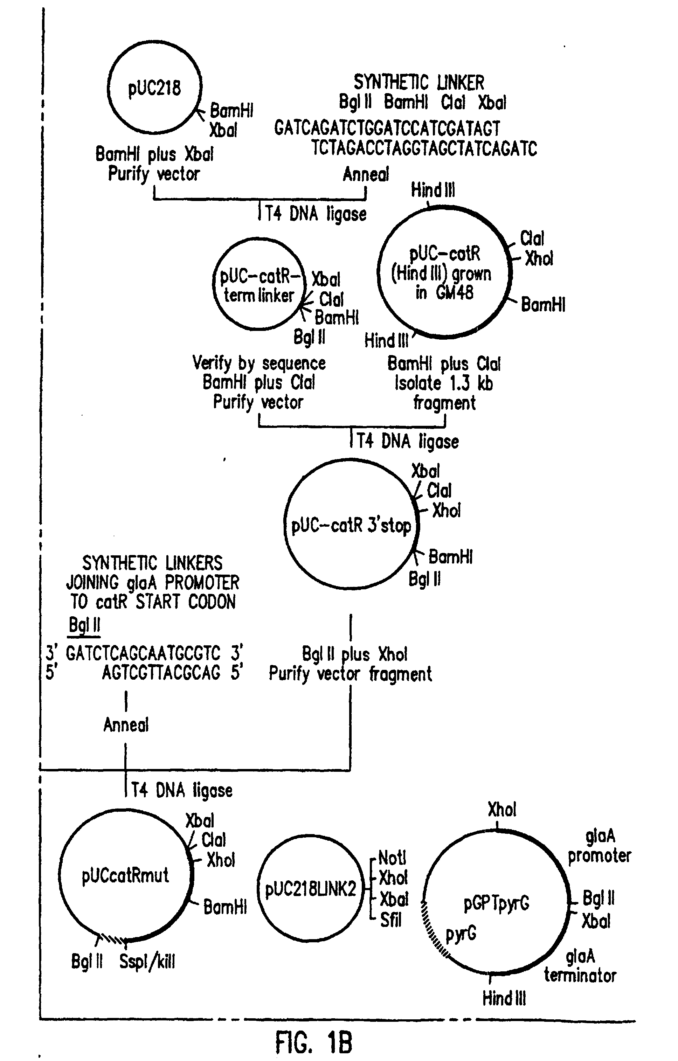

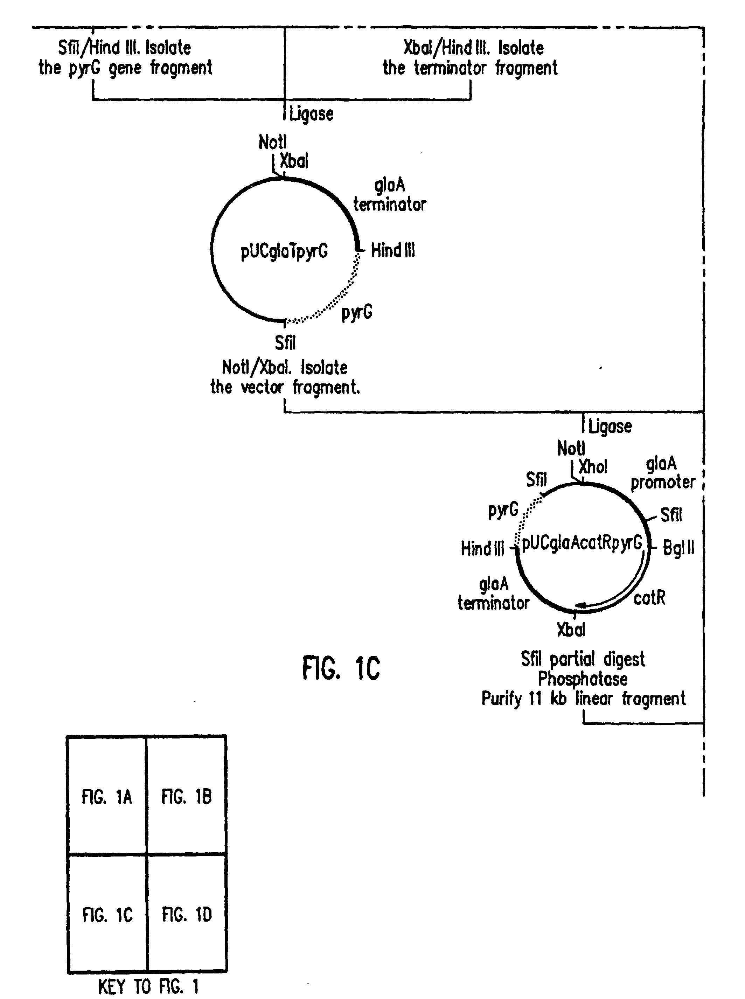

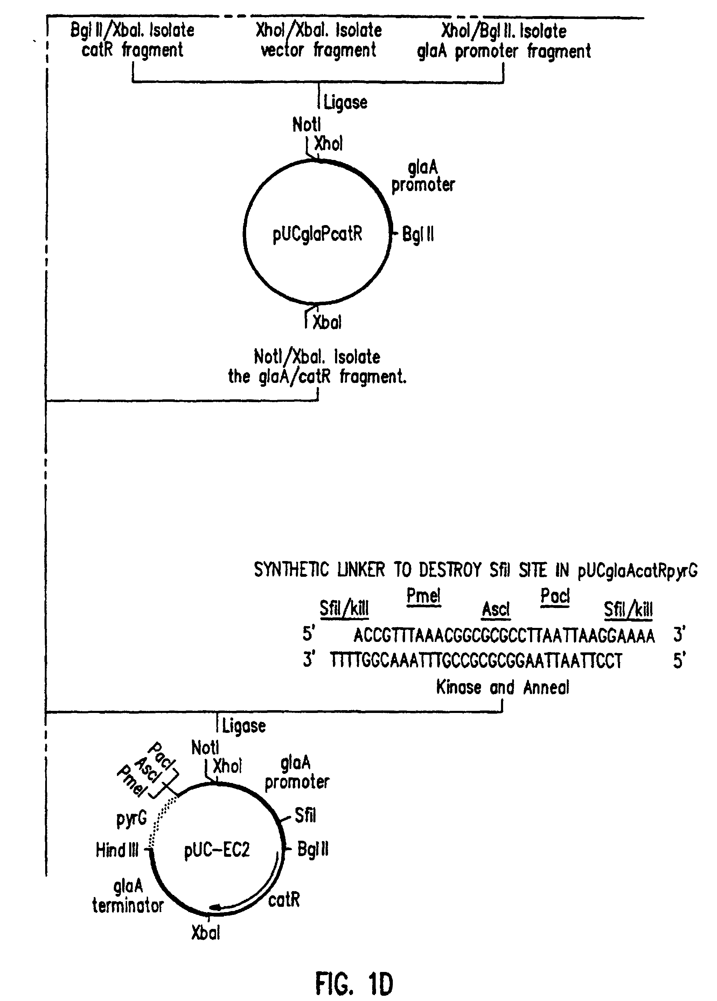

Figure 1 is a diagrammatic representation of the construction of the *catR* expression plasmid which contains the *A. niger glaA* promoter, *catR* coding region, *glaA* terminator and *A. niger pyrG* gene. A linear fragment (EC2L) containing these components was excised by digestion with *NotI* and *PmeI* and used to transform the host strain *A. niger ΔgoxA pyrG metC*.

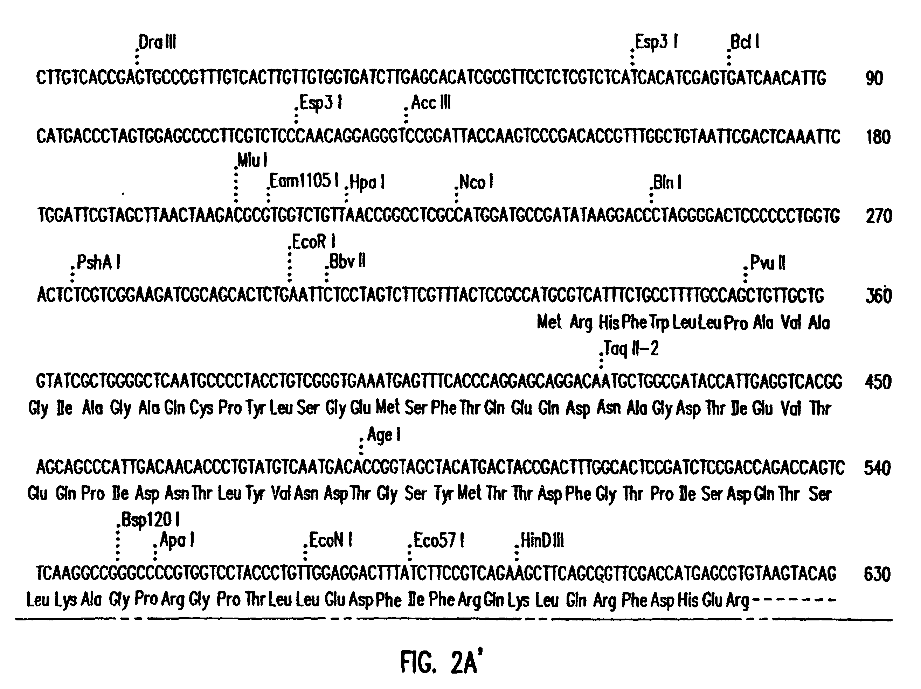

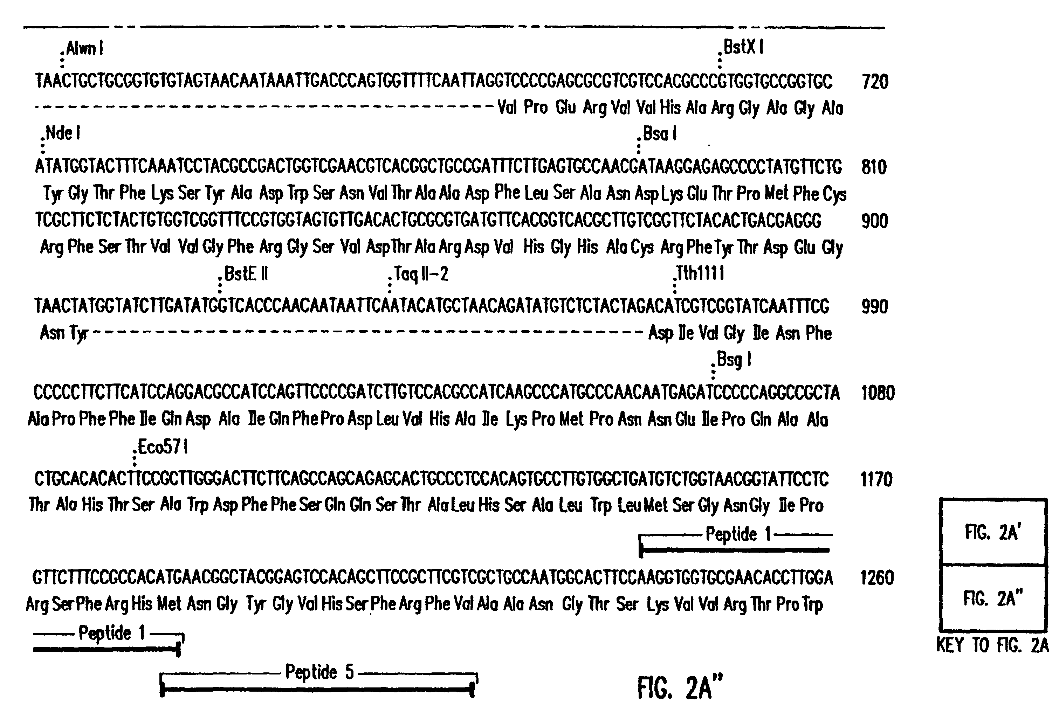

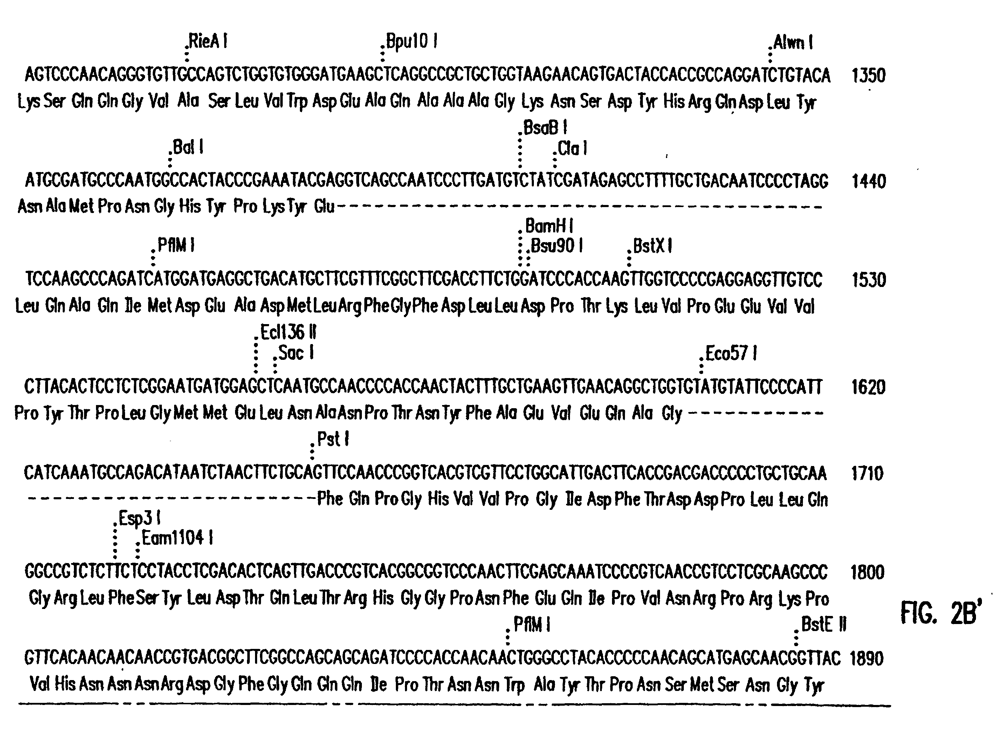

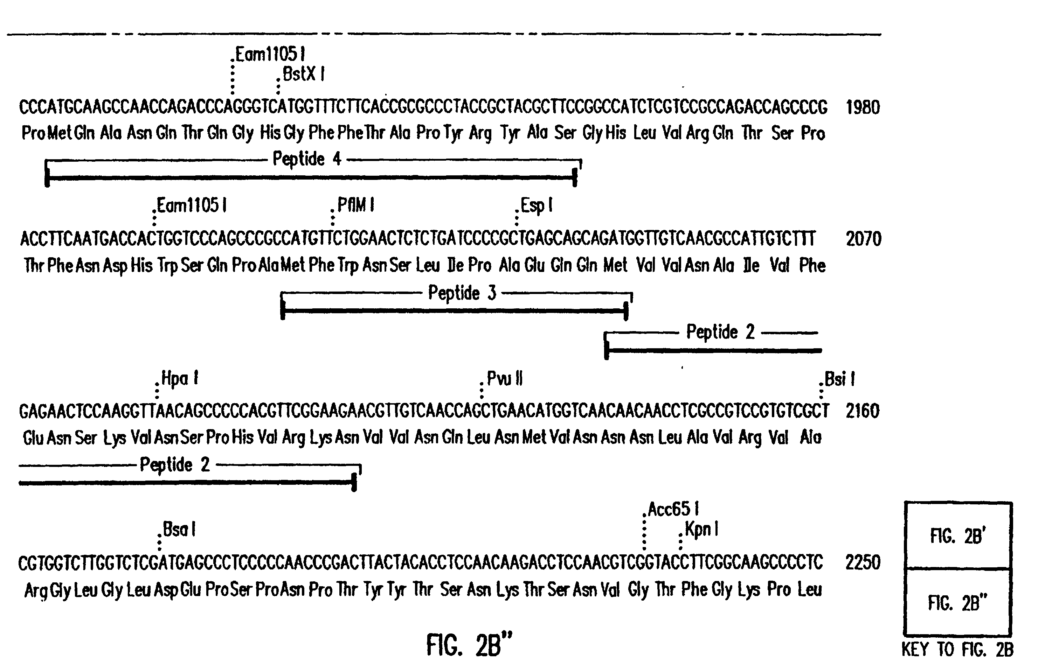

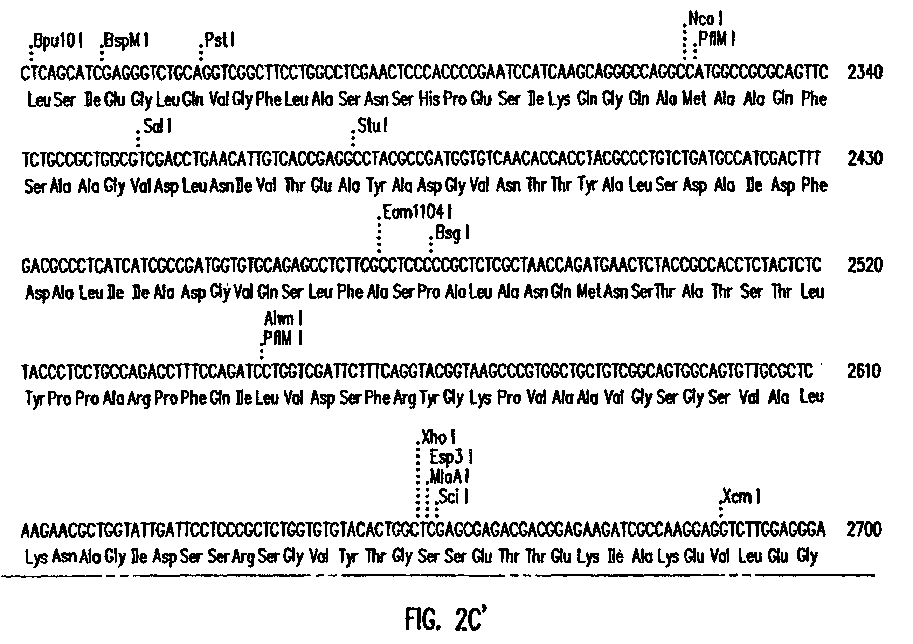

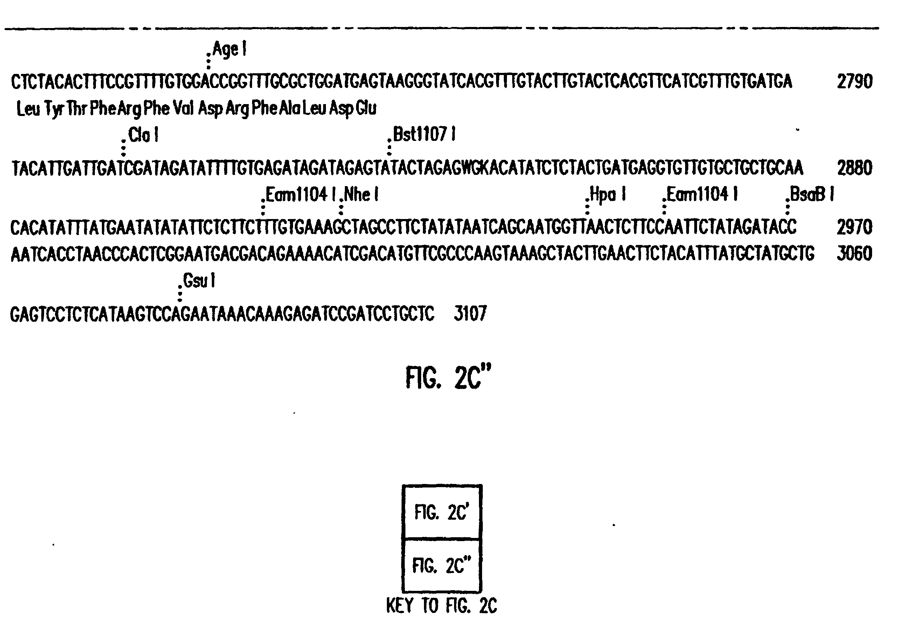

Figure 2 shows the nucleotide sequence and deduced amino acid sequence of the *A. niger catR* gene and flanking regions. Restriction sites for enzymes recognizing hexanucleotide and octanucleotide sequences are shown. Introns are denoted by dashed lines. Deduced amino acid sequences corresponding to peptides sequenced directly from the catalase-R protein are underlined with a solid bar.

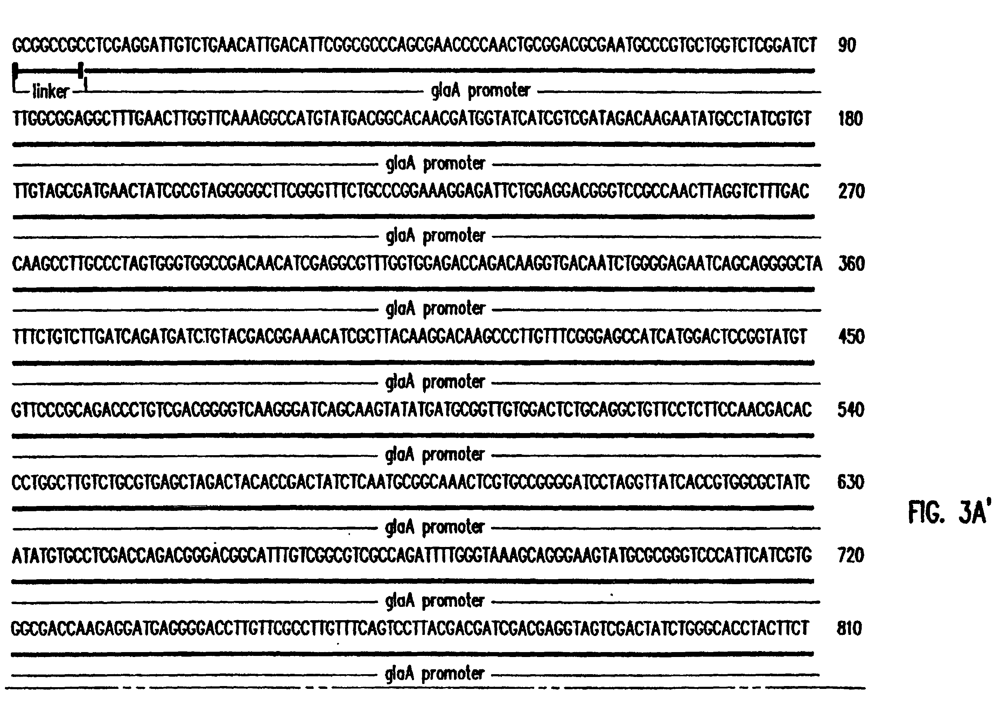

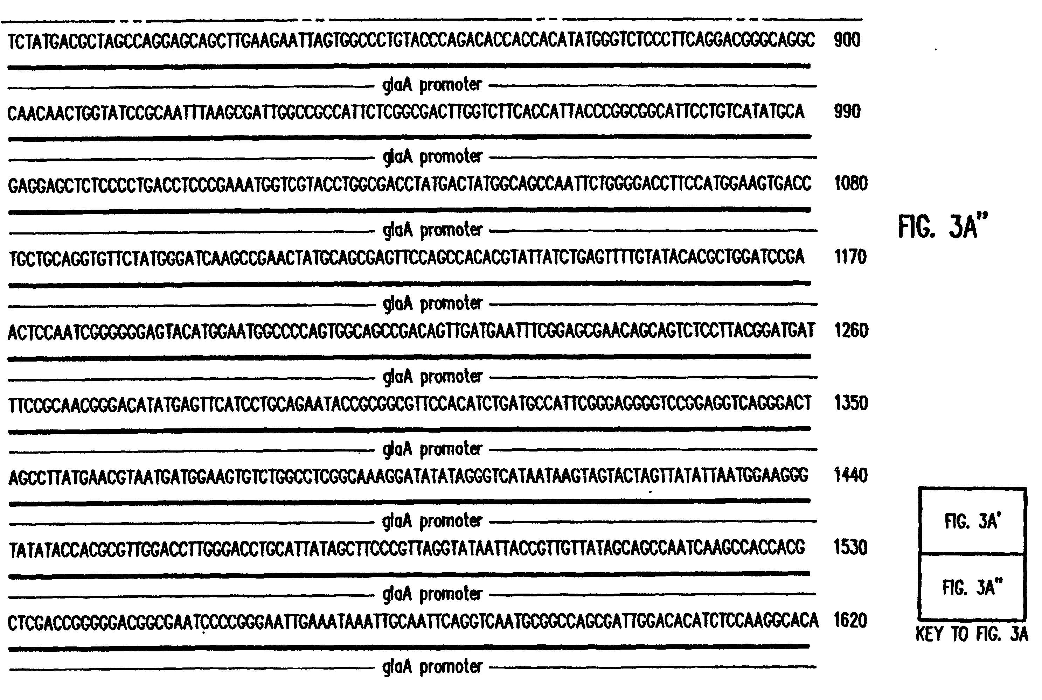

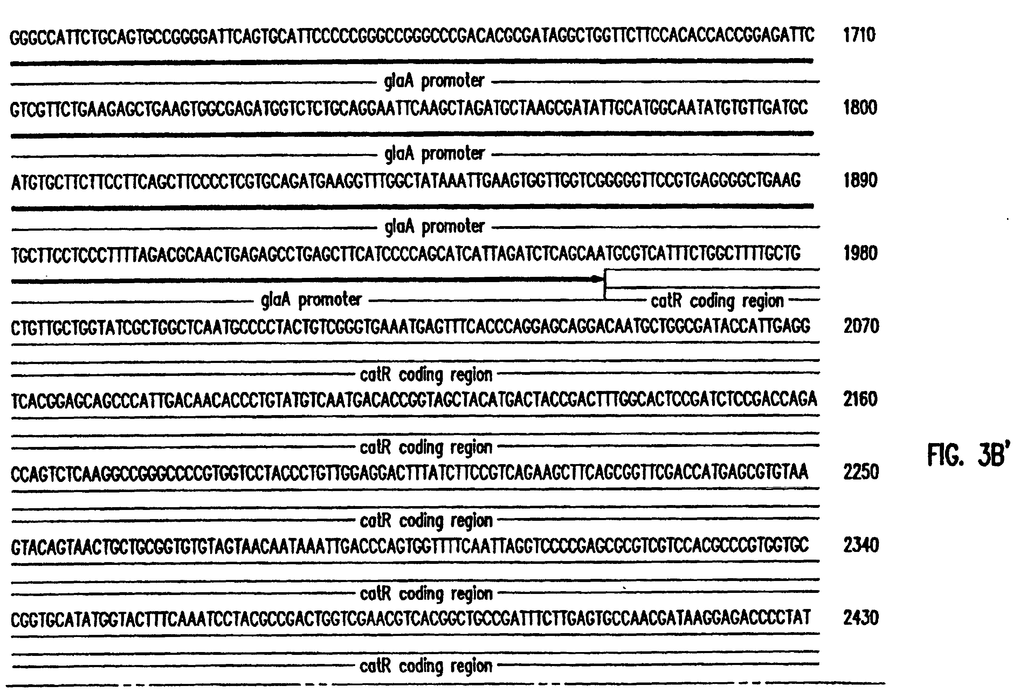

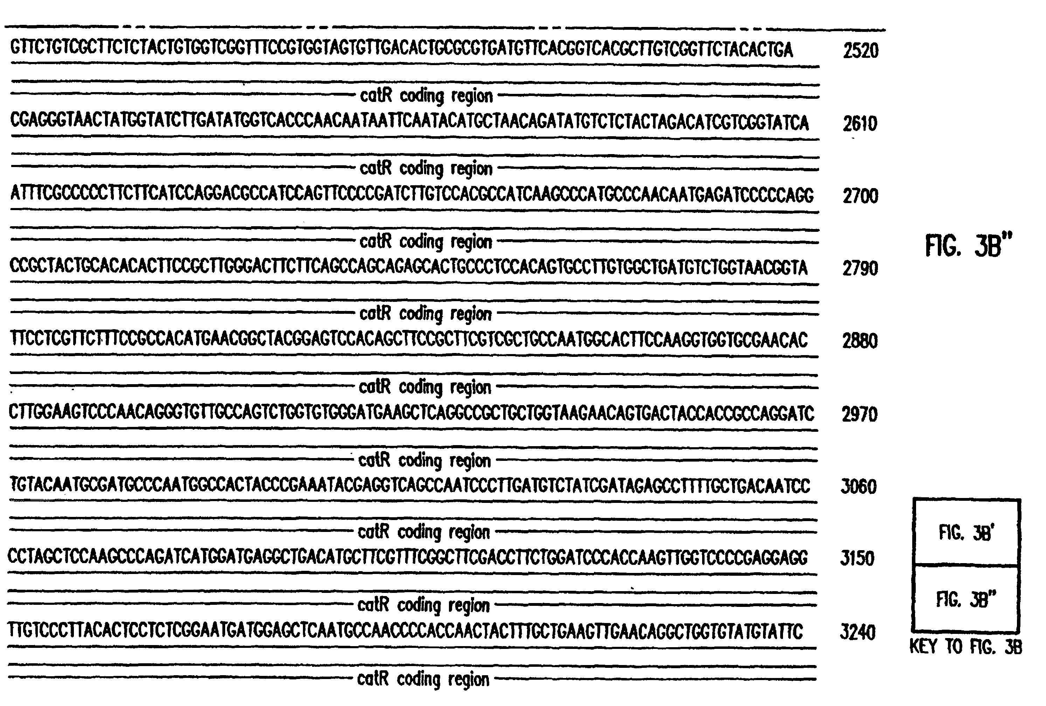

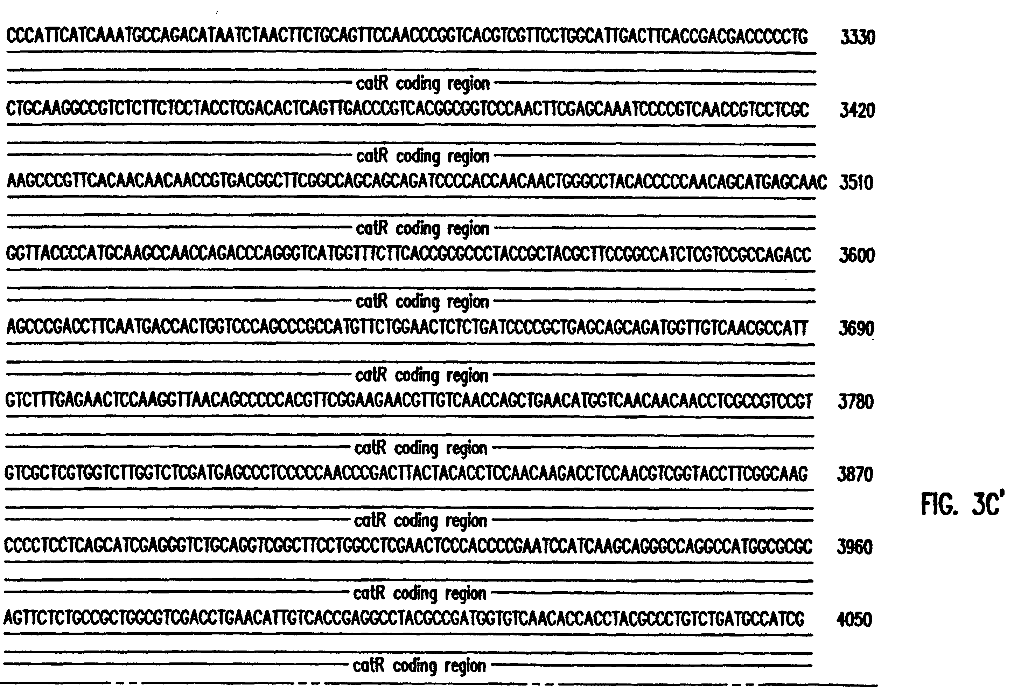

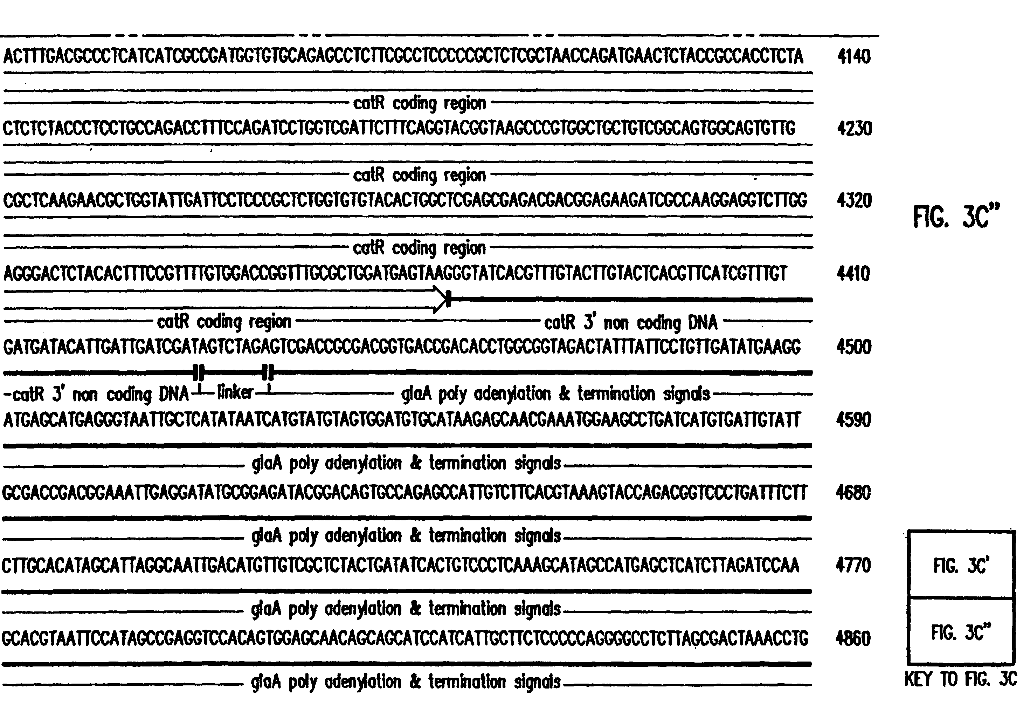

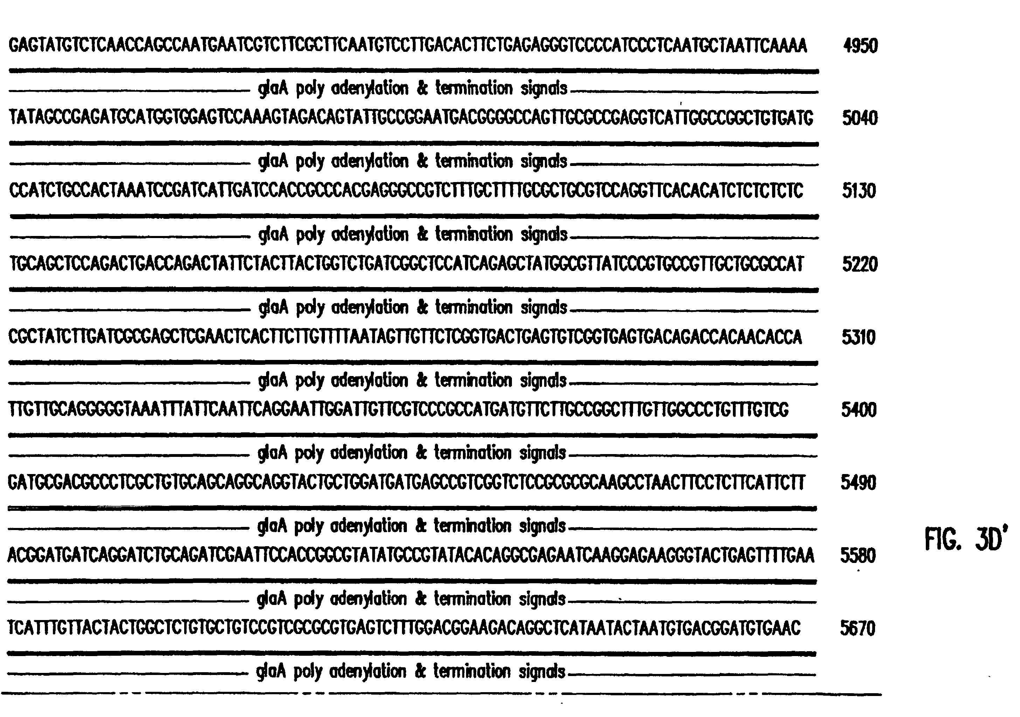

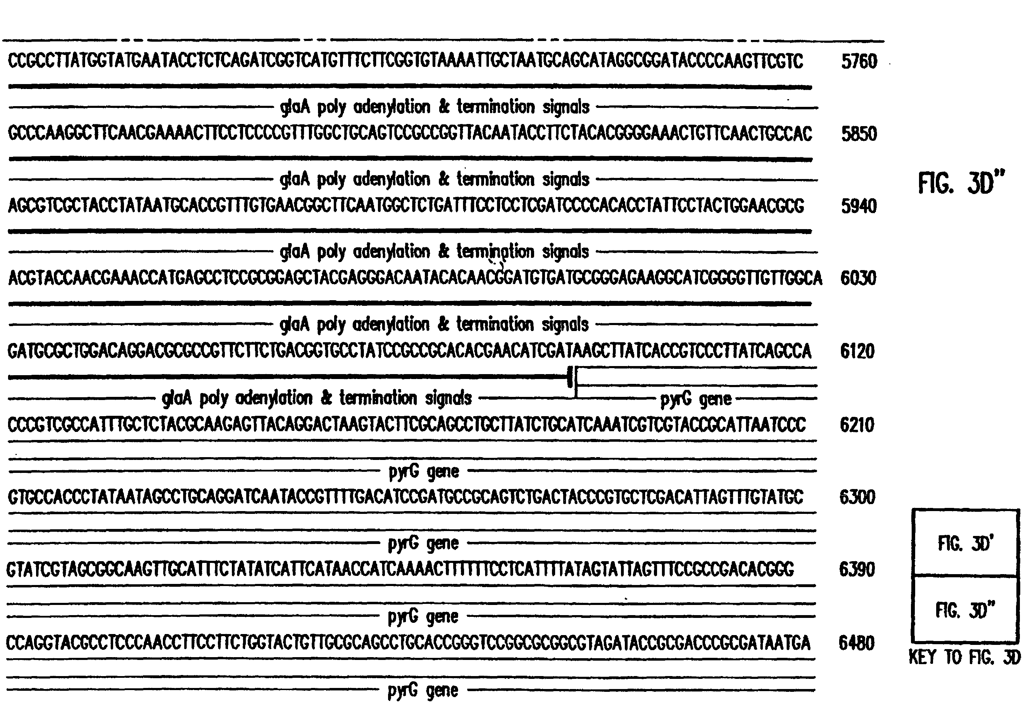

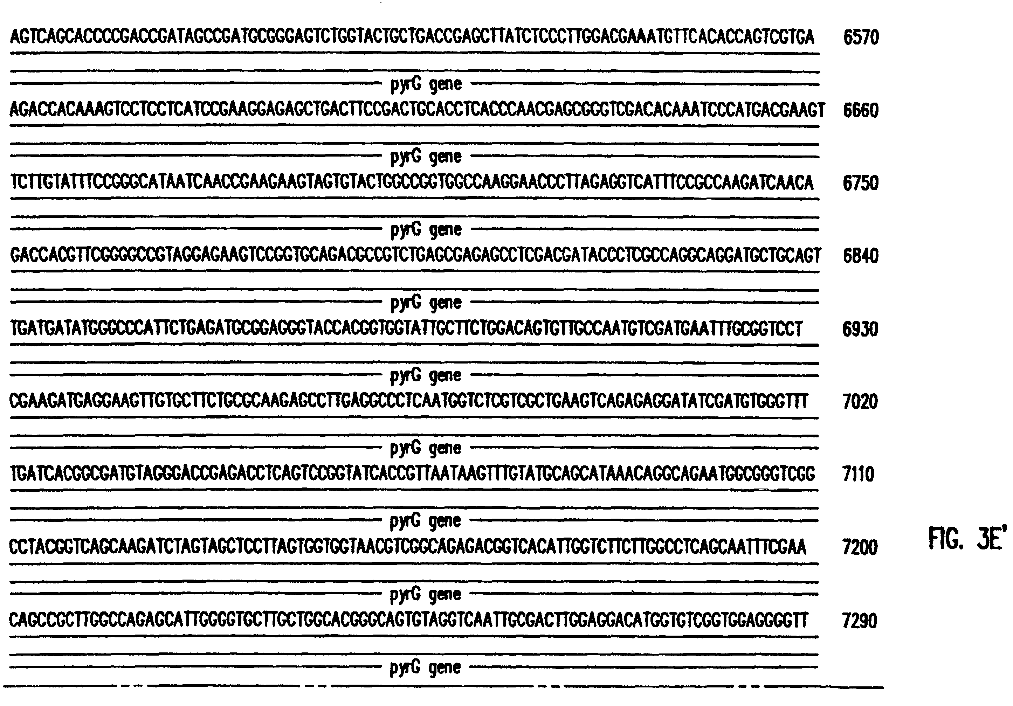

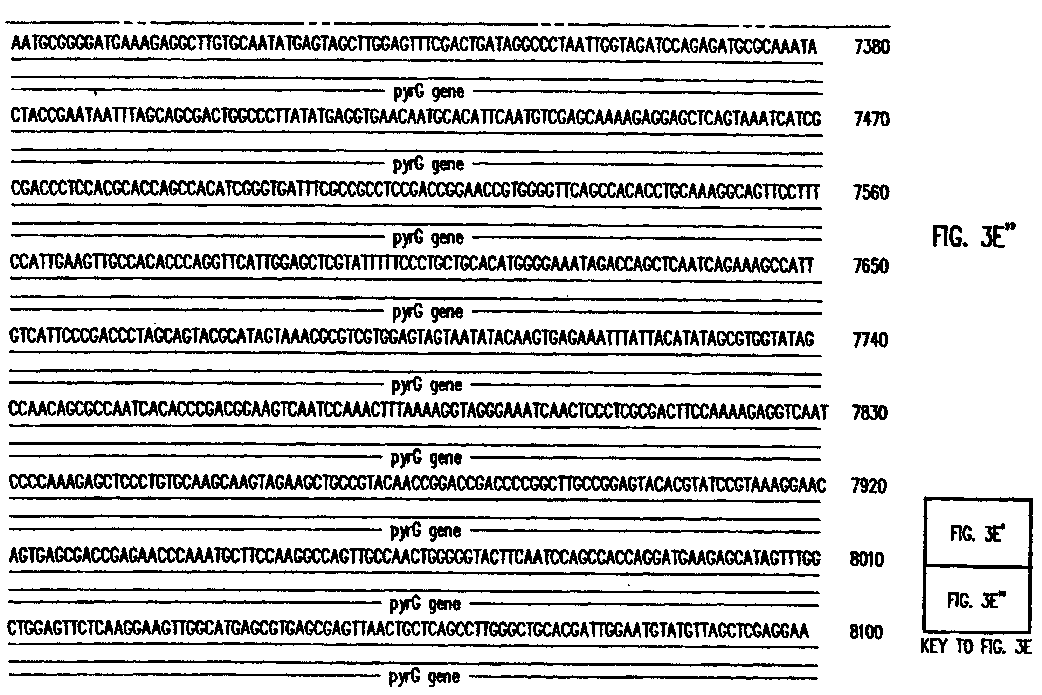

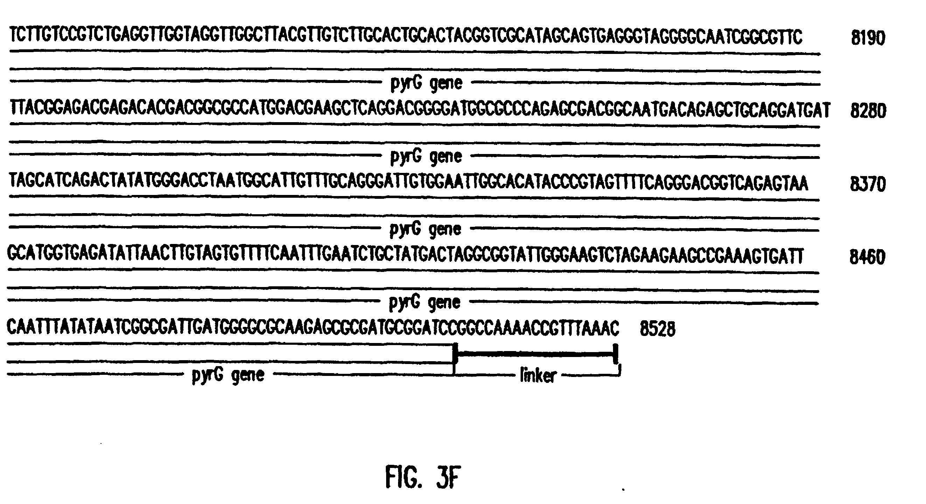

Figure 3 is the complete nucleotide sequence of the linear fragment (EC2L) used to transform *A. niger ΔgoxA pyrG metC*.

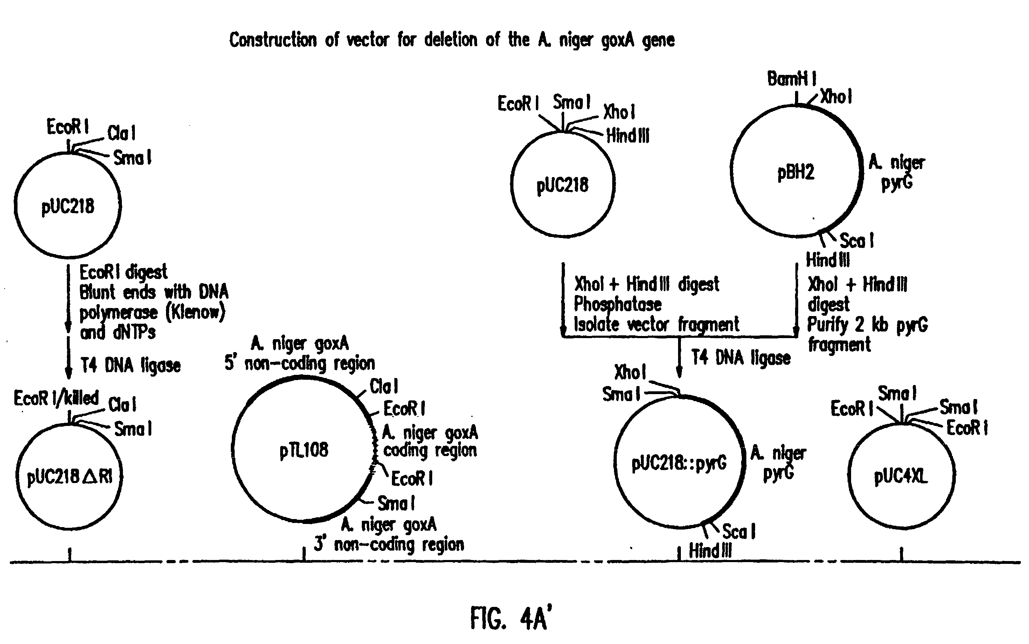

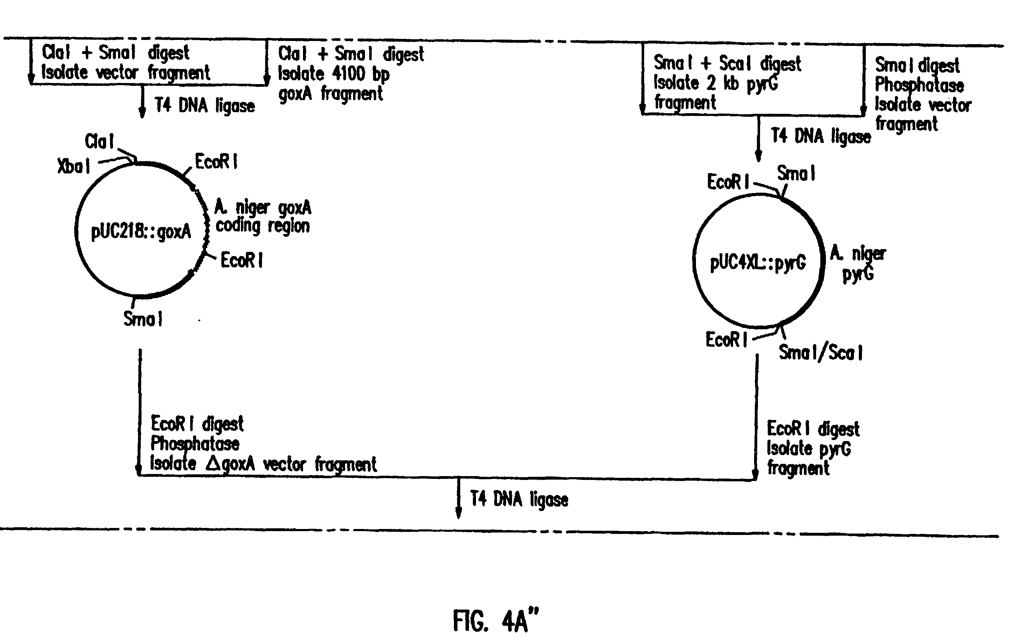

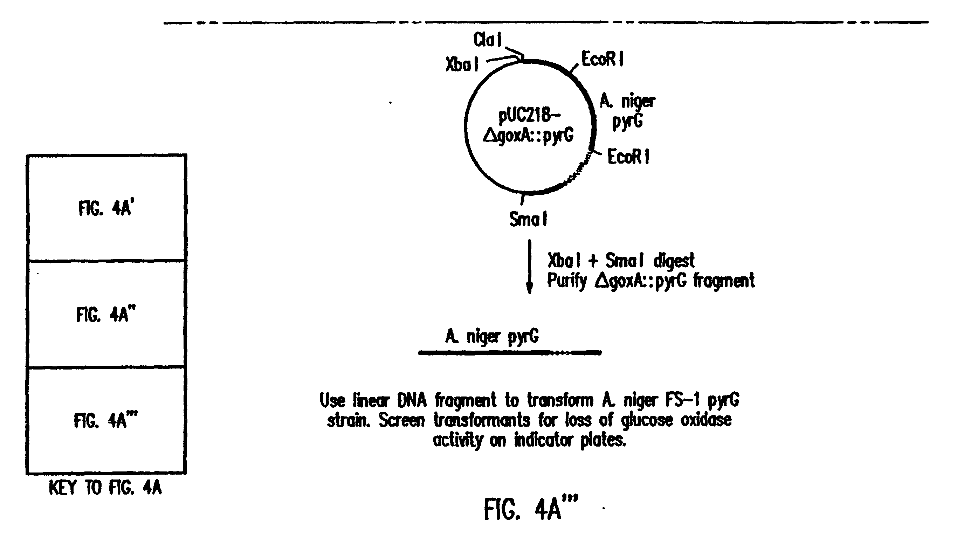

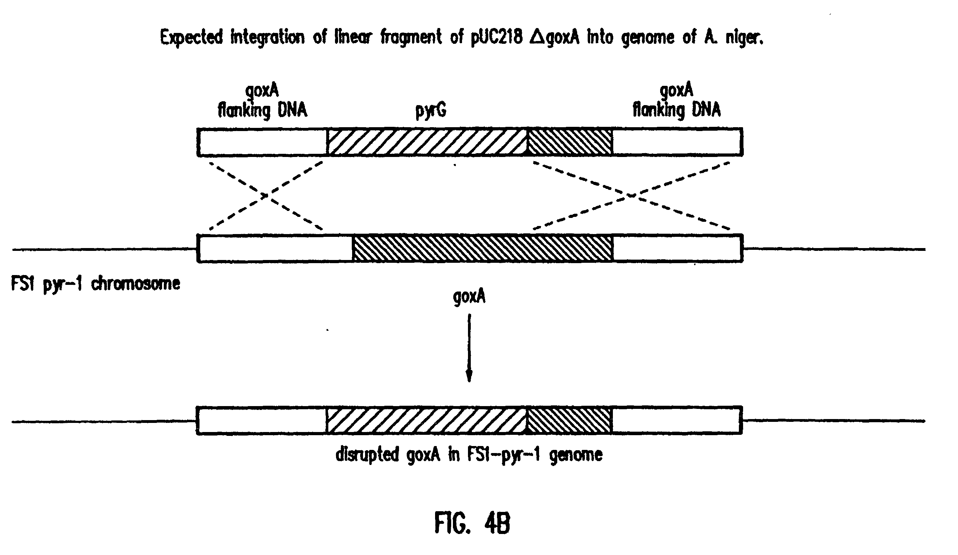

Figure 4, Panel A is a diagrammatic representation of the construction of the *A. niger* vector for deletion of the glucose oxidase (*goxA*) gene. A linear fragment comprising the *SmaI - ClaI* segment was excised and used to transform the host strain *A. niger pyrG.* Panel B is a schematic showing the expected integration event at the *goxA* locus which results in replacement of the *goxA* coding region with the *A. niger pyrG* gene.

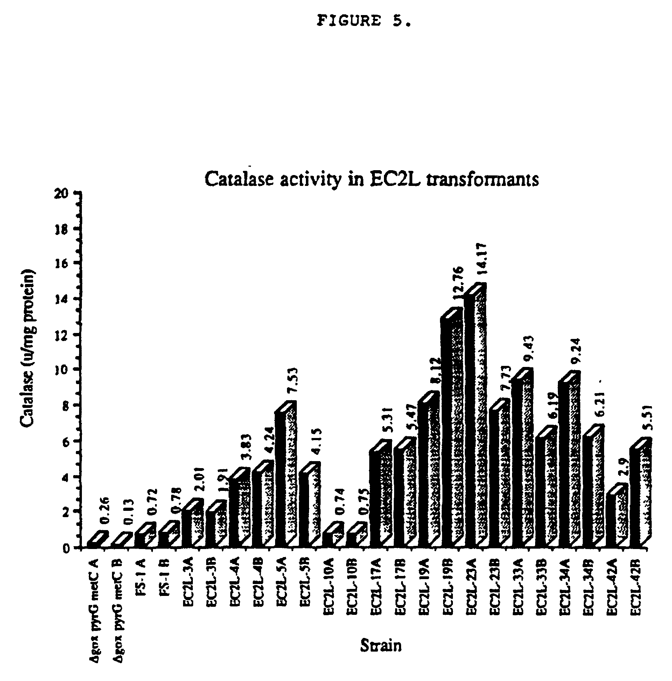

Figure 5 is a graph showing catalase production among strains of *A. niger ΔgoxA pyrG metC* transformed with the *catR* expression cassette (EC2L). The original parent strain, A. *niger* FS-1, and the host strain *A. niger ΔgoxA pyrG metC* are included as controls. Each strain was grown in duplicate and the assay results from each are shown.

### Description of the Preferred Embodiments.

The details of the catR expression vector construction and genetic modifications used to derive improved catalase production strains are described. One skilled in the art will understand that various changes in the following examples could be made. Accordingly, the examples are not intended to be limiting.

The techniques used in cloning the *A. niger catR* gene and construction of the *catR* expression cassette are conventional techniques described in Sambrook, et al., 1989 *Molecular Cloning, A Laboratory Manual,* Cold Spring Harbor Press, Cold Spring Harbor, NY.

### 1. Cloning and Characterization of the A. niger catR Gene.

Purified catalase-R was obtained from a commercial preparation of *A. niger* catalase (Fermcolase 1000, Genencor International, Inc.) and a series of proteolytic fragments were generated. These peptide fragments were subjected to amino acid sequence analysis. The amino acid sequence information was employed to design synthetic DNA probes for identification of *catR*-specific cDNA sequences contained within a λgt11 library. Briefly, the peptide fragment Met-Phe-Trp-Asn-Ser-Leu-Ile-Pro-Ala-Glu-Gln-Gln-Met was used to design a pool of three synthetic oligonucleotides having the following sequences:

5' ATG TTC TGG AAC AGC CTG ATC CCC GCC GAG CAG CAG ATG 3'
5' ATG TTC TGG AAC TCC CTG ATC CCC GCC GAG CAG CAG ATG 3'
5' ATG TTC TGG AAC AGC TTG ATC CCC GCC GAG CAG CAG ATG 3'

This peptide was chosen because the amino acids give minimally degenerate codon choices, i.e., the differences among the three synthetic oligonucleotides represent alternate codon choices where there was no strong bias in the known codon usage pattern for *A. niger.* This position of this proteolytic fragment corresponds to peptide 3 shown in Figure 2. A clone containing a partial cDNA fragment was positively identified by hybridization with the synthetic DNA probe and nucleotide sequence analysis of this clone confirmed that it encoded catalase-R. This cloned cDNA segment was used to probe a library of *A. niger* genomic DNA. Subsequently, the entire *catR* gene, plus upstream and downstream transcriptional control elements, was assembled as a 9.0 kb *Hind*III - *Xho*I restriction fragment. The nucleotide sequence of the *catR* coding region has been determined and is given in Figure 3.

### 2. Construction of a Catalase Expression Vector-Cassette (EC2) Used for Transformation of A. niger.

The *catR* expression vector used for these studies utilizes transcriptional and translational control signals from the well-characterized *A. niger* glucoamylase *(glaA)* gene. Unlike the *catR* promoter, the strong *glaA* promoter does not require H₂O₂ for induction. Instead, the *glaA* promoter responds to the presence of starch, maltose or other malto-oligosaccharides (Nunberg, et al., 1984 Mol. Cell. Biol. **4:** 2306-2316; Barton, et al., 1972 J. Bacteriol. **111:** 771-777; Fowler, et al., 1990 Curr. Genet. **18:** 537-545). Thus, use of the *glaA* promoter allows construction of catalase production strains which are not dependent on the generation of hydrogen peroxide for induction of catalase synthesis. Construction of the vector-cassette for expression of catalase under transcriptional control of the *glaA* promoter is outlined in Figure 1. The essential feature of this construct is that the glucoamylase-catalase expression unit (i.e., *glaA* promoter + *catR* coding region + *glaA* terminator) and the adjacent selectable marker (the *A. niger pyrG* gene) can be excised on a single *NotI - PmeI* restriction fragment (Figure 1).

The *catR* coding region was joined to the *glaA* promoter utilizing a synthetic oligonucleotide linker (13 base pairs) designed to couple these two DNA segments via a *BglII* site in the *glaA* promoter to a unique *SspI* site four base pairs after the *catR* start codon (introduced by site-directed mutagenesis). Insertion of this linker restores the nucleotide sequence of *catR* to that which existed prior to the site-directed mutagenesis and precisely fuses the *catR* coding region to the *glaA* promoter. In a description of the *glaA* promoter region given by Fowler, et al., (1990 Curr. Genet. **18:** 537-545) it was noted that there are DNA sequences far upstream of the start codon which are required for high level expression. These sequences, which presumably represent transcriptional enhancer elements, are included on the 1.9 kb *glaA* promoter segment included in construction of the *catR* expression cassette. Similarly, the *glaA* terminator region was linked to the 3'-end of catR via a naturally-occurring *ClaI* site downstream of the catalase-R gene stop codon. An *XbaI* site adjacent to *ClaI* was incorporated using a synthetic DNA linker and was then used to complete the terminator fusion. This terminator segment, which encodes information necessary for proper polyadenylation and termination of transcription, is the same segment as that which was used for Genencor's chymosin expression vector (Cullen, et al., 1987 Bio/Technol. **5:** 369-376). A restriction fragment containing the *A. niger pyrG* gene (Wilson, et al., 1988 Nucl. Acids Res. **16:** 2339) was subcloned adjacent to the *glaA* terminator such that the entire glucoamylase-catalase-selectable marker cassette was encoded on a single restriction fragment (the nucleotide sequence of this fragment (EC2L) is given in Figure 3).

### 3. Development of A. niger Strains to be Used in the Production of Catalase.

Features of the *A. niger* strain used as a host for expression of the glucoamylase-catalase cassette include a) uridine-requiring auxotrophy, specifically a *pyrG* auxotrophic mutation, b) deletion of the gene encoding glucose oxidase, *goxA*, and (c) a methionine-requiring auxotrophy, specifically mutation which renders the cells deficient in cystathionase (*metC*) activity. While the *metC* marker is not required for high level expression of catalase-R, it was included as a feature of the host strain to satisfy limited survivability regulation of government regulatory agencies. The catalase expression cassette described above was used to transform the *A. niger ΔgoxA pyrG metC* strain and the resulting transformants were screened in shake flask cultures for their ability to produce high levels of catalase. From these transformants, the highest catalase producers were selected for further study. Shake flask cultures were grown for two days at 33°C in 50 ml of a liquid medium that was made according to the following recipe: For each liter of medium add maltodextrin [Staley 200, A.E. Staley Co., (100 g)], ammonium sulfate (4 g), calcium chloride (0.4 g), magnesium sulfate (0.6 g), corn steep liquor [Archer Daniels Midland Co., (10 g)], and potassium phosphate (3 g); The volume is brought to 500 ml with distilled water, the pH is adjusted to 7.0, and the solution is autoclaved; Separately a 500 ml solution of 12% calcium carbonate is made in distilled water, the pH is adjusted to 7.0, and the solution autoclaved. The two sterile mixtures were combined aseptically to give one liter of catalase production medium. After two days growth, the mycelia were harvested by filtration (Miracloth, Calbiochem, Inc.), and the cells were rapidly frozen in liquid nitrogen. The cells were disrupted by grinding the frozen pellet in an electric coffee grinder for approximately 60 sec or until a fine powder was obtained. The disrupted cells were resuspended in an extraction buffer that contained 100 mM sodium formate, pH 7, 0.01% sodium dodecylsulfate, and 1 mM each of phenylmethyl sulfonyl fluoride and pepstatin. Insoluble debris was removed by centrifugation at approximately 1500 ¥g, and the activity of soluble catalase in the extract was measured by previously described methods (Patti and Bonet-Maury 1953 Bull Soc. Biol. **35:** 1177;
Teranishi, et al., 1974 Agric. Biol. Chem. **38:** 1213). Specific methods for generation of the catalase production organisms are outlined below. The parental strain for all studies described herein was *A. niger* FS-1 (NRRL3).

### Isolation of A. niger FS-1 pyrG Strains.

5-Fluoro-orotic acid (FOA), a toxic analog of orotic acid, has been used to select uridine-requiring auxotrophs in filamentous fungi and yeasts (VanHartingveldt, et al., 1987 Mol. Gen. Genet. **206:** 71-75). Fungal strains deficient in orotidine-5'-monophosphate decarboxylase (*pyrG* gene product), are resistant to FOA and require exogenous uridine for growth. The *A. niger pyrG* gene was cloned (Wilson, et al., 1988 Nucl. Acids Res. **16:** 2339) and used as a selectable marker for the transformation of *pyrG* mutant strains. An advantage of using FOA as a positive selection for *pyrG* auxotrophs is that spontaneous mutants can be selected without need for excessive mutagenesis and screening. The method of selecting *A. niger* FS-1 *pyrG* mutants is as follows: Spores of *A. niger* FS-1 were spread onto the surface of minimal medium plates containing 2 mg/ml uridine and 1.2 mg/ml FOA. Resistant colonies (FOA^{r}) were evident after 2-3 days growth at 37°C. Spores from six FOA^{r} colonies were streaked onto fresh medium containing FOA, and isolated colonies were picked for further analysis. Three of the six FOA^{r} strains were shown to require uridine for growth. To determine which of the uridine-requiring strains had a non-functional *pyrG* gene, each of the strains was tested for its ability to be transformed (i.e., complemented) with a plasmid containing the *A. nidulans pyrG* gene. Only one strain, FS-1 *pyrG1*, gave transformants (an approximate frequency of 10 transformants per µg DNA) indicating that it carried a *pyrG* mutation. This strain was used for all subsequent experimentation.

### Generation of A. niger FS-1 ΔgoxA Strains.

To generate a chromosomal deletion in the *goxA* gene, a vector was constructed which contained 5'- and 3'-flanking DNA sequences from the *goxA* gene and a selectable *pyrG* gene inserted in place of a portion of the *goxA* coding region (see Figure 4). For complete information regarding the nucleotide sequence of the *goxA* gene, consult Frederick, et al., 1990 J. Biol. Chem. **265:** 3793-3802 989 and Kriechbaum, et al., 1989 FEBS Lett. **255:**63-66. Briefly, a 4.1 kb *ClaI-SmaI* fragment comprising the *A. niger* FS-1 *goxA* gene was subcloned into a pUC218-derivative (from which the *Eco*RI site had previously been removed) to give pUC218*goxA*. The *A. niger pyrG* gene was isolated from pUC4XL as an *Eco*RI fragment having 27 bp and 16 bp of pUC4XL polylinker DNA at either end. The *goxA* coding region was subsequently removed by digestion with *Eco*RI and the remaining plasmid fragment was ligated with the *Eco*RI fragment containing the *A. niger pyrG* gene to create pUC218Δ*goxA*. From this plasmid a 4.75 kb *SmaI-XbaI* restriction fragment which contains 5'- and 3'-flanking regions of the *goxA* gene with part of the *goxA* coding sequence removed and replaced with a functional *pyrG* gene was isolated. Use of this fragment to transform *A. niger* FS-1 *pyrG1* with selection for uridine prototrophy resulted in the isolation of several strains which failed to give a blue color on glucose oxidase indicator plates (Witteveen, et al., 1990 Appl. Microbiol. Biotechnol. **33:** 683-686). Southern blotting analysis of genomic DNA extracted from these *goxA*-deficient transformants indicated that the *ΔgoxA::pyrG* cassette had integrated via a homologous recombination event at the *goxA* locus (as diagramed in Figure 4B). In other words, the selectable *pyrG* gene had replaced the *goxA* coding region.

As shown in Figure 5, catalase production in *ΔgoxA* mutants was approximately three- to six-fold lower than the parental strain FS-1. We interpret these data to indicate that in the absence of glucose oxidase little hydrogen peroxide is generated, and this in turn has an adverse effect on catalase induction.

### Isolation of A. niger FS-1 ΔgoxA pyrG Strains.

Spontaneous uridine-requiring mutants of *A. niger FS-1 ΔgoxA* were selected using FOA as described above. This step was necessary for subsequent transformation of the strain with the *pyrG*-based EC2 cassette.

### Isolation of an A. niger FS-1 ΔgoxA pyrG metC Strain.

In order to limit the survivability of a recombinant catalase production organism in the environment, a methionine-requiring auxotrophy was introduced in the following manner. Spores of *A. niger FS-1 ΔgoxA pyrG* were mutagenized with UV light (95% killing) and survivors were subjected to filtration enrichment in *Aspergillus* minimal medium. With this technique, unwanted prototrophs germinate and grow to form mycelia which can be removed by filtration. Auxotrophic cells cannot germinate or grow in minimal medium, and therefore pass through porous filters (e.g., Miracloth, Calbiochem, Inc.). After several rounds of filtration and growth, the remaining spores were plated onto complete medium. Colonies were patched from these plates onto minimal medium agar and to fresh complete medium plates. Those which grew on complete medium but not on minimal agar were auxotrophic. From the population of auxotrophs, one colony was identified which grew on minimal medium supplemented with methionine. Upon further testing, it was discovered that the strain was defective in a specific step of the methionine biosynthetic pathway. Growth was supported by the addition of either homocysteine or methionine, but not by either homoserine or cystathionine. Based on the known biosynthetic pathway for methionine, it appears that this methionine-requiring auxotroph was deficient in cystathionase activity, and thus, it was given the designation of *metC* by convention with other organisms.

### 4. Transformation of the A. niger FS-1 ΔgoxA pyrG metC Strain and Characterization of Catalase Overproducing Strains.

The catalase expression cassette (in linear form) was isolated following digestion of the pUC-EC2 plasmid with *PmeI* and *NotI* and purification of the EC2 fragment by preparative gel electrophoresis. The purified DNA fragment was then used to transform the *A. niger ΔgoxA pyrG metC* strain, and prototrophic transformants were screened in shake flask culture for their ability to produce catalase. From approximately fifty transformants screened in shake flasks, ten were identified that produced significantly higher catalase levels than control strains. These ten strains were re-evaluated in duplicate shake flask cultures, and the results of catalase activity assays are shown in Figure 5. Nine of the ten strains produced significantly higher levels of catalase-R than the parent strain FS-1. Two of the transformants (EC2L-19, EC2L-23) produced catalase yields in shake flask cultures that were roughly ten to fifteen times the level produced by *A. niger* FS-1, and these strains were chosen for testing under large scale production conditions. Fermentation experiments at the 10 liter and 50,000 liter scale have shown that catalase-R production from transformant EC2L-23 corresponds to the level of catalase-R expression seen in shake flask studies.

Furthermore, HPLC analyses of organic acids produced during fermentations of *A. niger* EC2L-23 and the parental strain FS-1 gave the following yields of sodium gluconate:

| Strain | sodium gluconate (mg/L) |
|---|---|
| FS-1 | >200,000 |
| EC2L-23 (run 27) | 48 |
| EC2L-23 (run 28) | 123 |

These data show a dramatic decrease in the production of sodium gluconate waste material by transformant EC2L-23.

## Claims

1. A gene sequence comprising the coding region of a gene encoding a catalase R. enzyme from Aspergillus comprising the amino acid sequence of Figure 2A-C and an *Aspergillus* glucoamylase promoter gene functionally attached to said gene.

2. The gene sequence according to claim 1 wherein the *Aspergillus* glucoamylase promoter is from *A. niger.*

3. The gene sequence according to claim 1 or claim 2 wherein the gene comprises the catR-sequence shown in Figure 2A-C or in Figure 3A-F.

4. A replicable plasmid or a vector capable of integration into an *Aspergillus niger* genome into which the gene sequence according to any one of claims 1-3 has been inserted.

5. An *Aspergillus niger* which has been transformed with a gene sequence according to any one of claims 1-4.

6. An *Aspergillus niger* according to claim 5 wherein the native glucose oxidase gene is deleted.

7. A method of producing a catalase R-enzyme from *Aspergillus* comprising the amino acid sequence of Figure 2A-C which comprises culturing an *Aspergillus niger* according to claim 5 or claim 6 using assimilable sources of carbon and nitrogen.

## Patentansprüche

1. Gensequenz, welche die kodierende Region eines Gens, das für ein Catalase R-Enzym von *Aspergillus* kodiert, welches die Aminosäuresequenz von Figur 2A-C umfasst, und ein *Aspergillus*-Glucoamylase-Promotorgen umfasst, das funktionell an dem Gen angebracht ist.

2. Gensequenz nach Anspruch 1, in der der *Aspergillus*-Glucoamylase-Promotor von *A. niger* abstammt.

3. Gensequenz nach Anspruch 1 oder Anspruch 2, in der das Gen die in Figur 2A-C oder in Figur 3A-F gezeigte catR-Sequenz umfasst.

4. Replizierbares Plasmid oder replizierbarer Vektor, das bzw. der zu einer Integration in ein *Aspergillus niger*-Genom in der Lage ist und in das bzw. den die Gensequenz nach irgendeinem der Ansprüche 1 - 3 inseriert worden ist.

5. *Aspergillus niger*, der mit einer Gensequenz nach irgendeinem der Ansprüche 1 - 4 transformiert worden ist.

6. *Aspergillus niger* nach Anspruch 5, in dem das native Glucoseoxidase-Gen deletiert ist.

7. Verfahren zur Erzeugung eines Catalase R-Enzyms von *Aspergillus,* das die Aminosäuresequenz von Figur 2A-C umfasst, umfassend das Kultivieren von *Aspergillus niger* nach Anspruch 5 oder Anspruch 6 unter Verwendung von assimilierbaren Quellen von Kohlenstoff und Stickstoff.

## Revendications

1. Séquence de gènes comprenant la région codante d'un gène codant pour une enzyme catalase-R à partir *d'Aspergillus* comprenant la séquence d'acides aminés de la figure 2A-C et un gène promoteur de glucoamylase *Aspergillus* fixé de manière fonctionnelle audit gène.

2. Séquence de gènes selon la revendication 1 dans laquelle le promoteur de glucoamylase d*'Aspergillus* provient de *A. niger*.

3. Séquence de gènes selon la revendication 1 ou la revendication 2 dans laquelle le gène comprend la séquence catR représentée sur la figure 2A-C ou sur la figure 3A-F.

4. Plasmide réplicable ou vecteur capable d'intégration dans un génome d'*Aspergillus niger* dans lequel la séquence de. gènes selon l'une quelconque des revendications 1 à 3 a été insérée.

5. *Aspergillus niger* qui a été transformé par une séquence de gènes selon l'une quelconque des revendications 1 à 4.

6. *Aspergillus niger* selon la revendication 5 dans lequel le gène de glucose oxydase native est supprimé.

7. Procédé de production d'une enzyme catalase-R en provenance d'*Aspergillus* comprenant la séquence d'acides aminés de la figure 2A-C qui comprend la mise en culture d'un *Aspergillus niger* selon la revendication 5 ou la revendication 6 en utilisant des sources assimilables de carbone et d'azote.
